# FASCICULE DE BREVET EUROPEEN

(11) **EP 2 419 438 B1**
(45) Date de publication et mention de la délivrance du brevet: **15.07.2015**
(21) Numéro de dépôt: 10718238.8
(22) Date de dépôt: 15.04.2010
(51) Int. Cl.: C07K 5/103, C07K 7/06, C07K 14/395, C07K 14/415, A61K 38/01, A61K 8/64, A61P 17/00, A61Q 17/04, A61Q 17/00, A61Q 19/08, A61K 38/07, A61K 38/08, A61K 8/97, A61Q 19/00

(54) **COMPOSITION COSMÉTIQUE ET/OU PHARMACEUTIQUE COMPRENANT UN HYDROLYSAT PEPTIDIQUE CAPABLE DE RENFORCER LA FONCTION BARRIÈRE**
EIN PEPTIDHYDROLYSAT, DAS DIE BARRIEREFUNKTION UNTERSTÜTZEN KANN, ENTHALTENDE KOSMETISCHE UND/ODER PHARMAZEUTISCHE ZUSAMMENSETZUNG
COSMETIC AND/OR PHARMACEUTICAL COMPOSITION COMPRISING A PEPTIDIC HYDROLYSATE THAT CAN REINFORCE THE BARRIER FUNCTION

(30) Priorité: 15.04.2009 FR 0901822
(43) Date de publication de la demande: 22.02.2012
(73) Titulaire: ISP Investments Inc., Wilmington, DE 19805 (US)
(72) Inventeur: DAL FARRA, Claude, Kerhonkson New York 12446 (US); DOMLOGE, Nouha, F-06560Valbonne (FR); BOTTO, Jean-Marie, F-06560 Valbonne (FR)
(74) Mandataire: Macquet, Christophe
(86) Numéro de dépôt international: PCT/FR2010/000311
(87) Numéro de publication internationale: WO 2010/119191

(56) Documents cités:
- WO-A-2004/096168
- FR-A- 2 904 552
- FR-A- 2 911 779
- FR-A- 2 915 384
- FR-A- 2 925 326
- FR-A- 2 925 327
- FR-A- 2 925 330

## Description

La présente invention se situe dans le domaine cosmétique et pharmaceutique, et plus particulièrement dans le domaine de la dermatologie. L'invention concerne un hydrolysat peptidique enrichi en peptide bioactif, capable de renforcer la fonction barrière cutanée et de stimuler la différenciation épidermique. Le peptide bioactif est caractérisé en ce qu'il comprend de 4 à 6 acides aminés dont au moins un résidu glycine, un résidu leucine et un résidu acide glutamique et possédant une séquence de formule générale (I) X₁-[Gly,Glu,Leu]-X₂-X₃ choisie parmi les séquences SEQ ID n°1 à SEQ ID n°7, provenant de l'hydrolyse de protéines de plantes choisies parmi l'épeautre, la pomme de terre, le maïs, le pois, ou de protéines de levures du genre Saccharomyces, et composé de peptides de poids moléculaire inférieur à 6 kDa. L'invention est également relative à une composition comprenant, dans un milieu physiologiquement acceptable, un hydrolysat peptidique enrichi en peptide bioactif en tant que principe actif capable de renforcer la fonction barrière de l'épiderme. L'invention est également relative à une composition pharmaceutique comprenant ce nouveau principe actif à titre de médicament. L'invention concerne enfin l'utilisation cosmétique dudit hydrolysat peptidique, en tant que principe actif pour activer la HMG-CoA réductase et ainsi renforcer la fonction barrière cutanée et stimuler la différenciation épidermique. L'invention porte encore sur un procédé de traitement cosmétique destiné à prévenir et/ou à lutter contre les agressions extérieures et les manifestations du vieillissement cutané.

La fonction première de l'épiderme est de constituer une barrière entre l'environnement extérieur et le milieu intérieur. C'est la couche la plus externe de l'épiderme, *le stratum corneum,* qui assure cette fonction. Il est composé de kératinocytes au stade ultime de leur différenciation, les cornéocytes, scellés les uns aux autres par un épais ciment intercellulaire, à la fois souple et imperméable. On distingue ainsi dans le *stratum corneum* un compartiment cellulaire, constitué des cornéocytes et un compartiment extracellulaire principalement constitué de lipides, organisés en structures multilamellaires.

Le contenu lipidique du *stratum corneum* humain est estimé à 15 % d'ester de cholestérol, 16 % d'acides gras libres saturés à longues chaînes, 32 % de cholestérol, 37 % de céramides, bien que les variations inter-individuelles soient assez importantes (Norlen L. et al. J. Invest. Dermatol. 1999; 112(1) p. 72-77). Ces lipides sont synthétisés par les kératinocytes des couches intermédiaires de l'épiderme, et sécrétés dans des organites spécialisés appelés « corps lamellaires » ou corps de Odland. En particulier, l'épiderme est un site très actif pour la synthèse du cholestérol. L'étape limitante de cette synthèse, et la plus finement régulée, est la conversion du 3-hydroxy-3-methylglutaryl-Coenzyme A (HMG-CoA) en mévalonate. Cette étape est catalysée par une enzyme membranaire dénommée HMG-CoA-réductase (E.C. 1.1.1.34). Les données de séquençage du génome humain montrent qu'il existe au moins 2 isoformes de HMG-CoA réductase, codées par un gène unique, localisé sur le chromosome 5 (Luskey et al., J Biol Chem., 1985 260(18), p.10271-7).

Dans la peau, le cholestérol joue un rôle dans la fluidité des membranes et en particulier, il semble assurer la mobilité des chaînes hydrocarbonées dans les bicouches lipidiques (Martini M.C., Pathol. Biol. 2003, (51), p. 267-270). Ainsi, en situation physiologique, le cholestérol est synthétisé à un niveau nécessaire pour maintenir une homéostasie. En revanche, à la suite d'une altération brutale de la barrière cutanée, on observe une augmentation importante et rapide de la synthèse de cholestérol, associée à une augmentation de l'expression et de l'activité de la HMG-CoA réductase (Menon G.K. et al., J. Lipid, Res., 1985, (26), P. 418-427).

Le rôle clé de l'HMG-CoA réductase en a fait une cible de choix pour moduler l'expression du cholestérol dans l'organisme. On a ainsi développé une classe de composés pharmacologique, dénommés statines destinés à inhiber l'HMG-CoA réductase dans le but d'abaisser le cholestérol circulant. Cet effet inhibiteur des statines se manifeste également dans la peau humaine. En effet, l'administration expérimentale de statines par voie topique perturbe la fonction barrière de la peau (Proksch E. et al., British J. Dermatol., 1993, (128), p. 473-482). Ces résultats confirment l'importance du cholestérol dans la fonction barrière épidermique et le rôle central de l'HMG-CoA réductase dans la modulation de sa synthèse.

Au cours du vieillissement cutané, l'intégrité de la barrière cutanée ainsi que ses capacités de réparation s'altèrent. On observe une déficience globale en lipides, aboutissant à une diminution des multicouches lipidiques du compartiment extracellulaire du *stratum corneum.* Ces changements fonctionnels sont en corrélation avec une susceptibilité accrue des peaux âgées aux agressions extérieures (Ghadially R. et al., J Clin Invest., 1995 (95 (5), p. 2281-90).

Indépendamment du vieillissement intrinsèque ou photo-induit, des altérations de la barrière cutanée peuvent se produire lors d'agressions extérieures.

Dans ce contexte, il est souhaitable de chercher à prévenir l'altération ou à rétablir la fonction barrière de l'épiderme. Dans ce domaine particulier, l'apport direct de substituts lipidiques, tels que les céramides (EP 1272148, US2007576937) ou certains dérivés du cholestérol (FR 2 789 312) a largement été décrit. D'autre part, l'utilisation d'huiles végétales pour activer la synthèse des lipides cutanés a également été décrite (EP 1 707 189). Dans le domaine de la cosmétique, le ciblage moléculaire de l'HMG-CoA réductase a déjà été exploité mais dans le but d'inhiber cette enzyme-clé, par exemple en utilisant des statines, déjà connues pour leurs propriétés inhibitrices de HMGCOA, dans le but d'obtenir un effet antivieillissement, (EP 0738510). Toutefois, à ce jour, aucun document ne décrit ou ne suggère l'objet de l'invention ; c'est-à-dire qu'un hydrolysat peptidique enrichi en peptide bioactif selon l'invention peut avoir des propriétés intéressantes pour renforcer la fonction barrière cutanée et pour stimuler la différenciation épidermique. Il est également possible d'améliorer par cette action certains dysfonctionnements pathologiques liés à la fonction barrière (peaux hypersensibles, irritées, ou réactives, eczéma atopique).

Le premier objet de la présente invention est un hydrolysat peptidique activateur de la HMG-CoA réductase, caractérisé en ce qu'il provient de l'hydrolyse de plantes choisies parmi l'épeautre (*Triticum monococum*), la pomme de terre (*Solanum tuberosum*); le maïs (*Zea mayz L.*), le pois (*Pisum sativum*) ou de levures du genre *Saccharomyces,* et plus particulièrement de l'espèce *Saccharomyces cerevisiae,* est composé de peptides de poids moléculaire inférieur à 6 kDa et est enrichi en peptide bioactif capable de renforcer la fonction barrière de l'épiderme, caractérisé en ce qu'il comprend de 4 à 6 acides aminés dont au moins un résidu glycine, un résidu leucine et un résidu acide glutamique et possédant une séquence de formule générale (I) :

X₁-[Gly,Glu,Leu]-X₂-X₃

dans laquelle,
X₁ est l'alanine, la valine, l'isoleucine ou aucun acide aminé,
X₂ est la serine ou la thréonine,
X₃ est la leucine, l'isoleucine ou aucun acide aminé,
choisie parmi les séquences :
(SEQ ID n°1) Ala-Glu-Gly-Leu-Ser-Ile
(SEQ ID n°2) Leu-Gly-Glu-Ser-Leu
(SEQ ID n°3) Val-Gly-Glu-Leu-Thr
(SEQ ID n°4) Ile-Gly-Glu-Leu-Ser
(SEQ ID n°5) Ala-Gly-Glu-Leu-Ser
(SEQ ID n°6) Gly-Glu-Leu-Thr-Ile
(SEQ ID n°7) Gly-Glu-Leu-Ser.

Les inventeurs ont en effet mis en évidence une activité cosmétique et thérapeutique, notamment dermatologique, d'hydrolysats peptidiques contenant certains peptides particuliers, dénommés peptides bioactifs ci-après.

Il ont en particulier mis en évidence que l'hydrolysat peptidique, enrichi en peptide bioactif, lorsqu'il est appliqué sur la peau, renforce la fonction barrière de l'épiderme et stimule la différenciation épidermique. Ces propriétés ont été démontrées par une meilleure protection du tissu cutané vis-à-vis des agressions extérieures et une augmentation de la production de lipides constitutifs de la couche cornée.

On entend par « peptide bioactif » selon l'invention un enchaînement d'au moins quatre acides aminés, liés entre eux par des liaisons peptidiques ou par des liaisons peptidiques modifiées et qui possède une activité *in vivo* ou *in vitro* caractéristique de l'activité du principe actif selon l'invention.

L'activité biologique caractéristique selon l'invention est définie *in vitro* par la capacité du peptide à activer l'HMG-CoA réductase, soit par l'augmentation de la synthèse protéique de la HMG-CoA réductase (par modulation directe ou indirecte de l'expression génique de la HMG-CoA réductase), soit par l'augmentation de l'activité enzymatique de la HMG-CoA réductase, soit par d'autres processus biologiques tels que la stabilisation de la protéine HMG-CoA réductase ou encore la stabilisation des transcrits d'ARN messager.

On entend par peau, l'ensemble des tissus de recouvrement constituant la peau et les muqueuses, y compris les phanères (cheveux, cils, poils, sourcils).

On entend par « hydrolysat peptidique », un mélange de composés majoritairement représentés par des peptides ou des oligopeptides. Selon l'invention, on utilisera indifféremment les termes « hydrolysat peptidique » ou « principe actif ».

On entend par « composés de nature peptidique », les fragments de protéines, les peptides et les acides aminés libres présents dans l'hydrolysat peptidique selon l'invention.

On entend par « application topique », le fait d'appliquer ou d'étaler le principe actif selon l'invention, ou une composition le contenant, à la surface de la peau ou d'une muqueuse. On entend par « physiologiquement acceptable », que l'hydrolysat peptidique selon l'invention, ou une composition le contenant, est approprié pour entrer en contact avec la peau ou une muqueuse sans provoquer de réactions de toxicité ou d'intolérance.

Selon un mode de réalisation particulièrement intéressant, le peptide biologiquement actif correspond à la séquence SEQ ID n°5.

Le principe actif selon l'invention peut être obtenu par extraction de protéines d'origine végétale ou de levure, suivie d'une hydrolyse contrôlée qui libère des composés de nature peptidique, parmi lesquels se trouvent les peptides bioactifs.

L'utilisation d'hydrolysats peptidiques, et en particulier d'hydrolysats peptidiques de bas poids moléculaires, présente de nombreux avantages en cosmétique. Outre le fait de générer des composés de nature peptidique qui ne préexistaient pas dans le mélange protéique de départ, l'hydrolyse et la purification permettent d'obtenir des mélanges plus stables, plus facilement standardisables et ne provocant pas de réactions allergiques en dermato-cosmétique.

Il est également possible d'utiliser certains extraits hydrolysés sans en purifier les composés de nature peptidique correspondant aux peptides bioactifs selon l'invention, mais en s'assurant toutefois de la présence desdits peptides par des moyens analytiques appropriés.

De très nombreuses protéines trouvées dans les plantes et les levures sont susceptibles de contenir des peptides bioactifs au sein de leur structure. L'hydrolyse ménagée permet de dégager ces composés de nature peptidique particuliers. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les composés de nature peptidique ensuite.

Ledit principe actif provient de l'hydrolyse de protéines de plantes choisies parmi l'épeautre, la pomme de terre, le maïs, le pois, ou de protéines de levures de l'espèce saccharomyces. De préférence, les plantes utilisées ne sont pas soumises à une fermentation préalable.

Ainsi, l'invention peut être réalisée en utilisant des graines d'engrain ou petit épeautre (*Triticum monococcum*), qui est un blé diploïde très ancien contenant un taux particulièrement élevé en protéines (Vallega 1992).

L'invention peut également être réalisée en utilisant des tubercules de pomme de terre du genre Solanum et plus particulièrement de l'espèce *Solanum tuberosum.* Le tubercule n'appartient pas à la racine de la plante, mais à sa tige enterrée, dont partent des rameaux plus grêles appelés rhizomes, à l'extrémité desquels se forment les tubercules.

L'invention peut également être réalisée en utilisant les graines d'une des nombreuses plantes du genre Zea et préférentiellement l'espèce *Zea mays L.* Selon l'invention le matériel végétal utilisé sera le grain et préférentiellement le grain débarrassé de son enveloppe par une étape de décorticage.

L'invention peut également être réalisée en utilisant l'une des nombreuses plantes de la famille des pois (Fabacées). Selon l'invention on utilise les plantes de l'espèce des pois *Pisum sativum L.* Le terme pois désigne aussi la graine, elle-même riche en protéines (25 %). Selon l'invention, le matériel végétal utilisé sera le grain et préférentiellement le grain débarrassé de son enveloppe par une étape de décorticage.

L'invention peut également être réalisée en utilisant des levures du genre Saccharomyces ; et d'une manière préférentielle de l'espèce *Saccharomyces cerevisiae.*

Toute méthode d'extraction ou de purification connue de l'homme du métier peut être utilisée afin de préparer l'hydrolysat selon l'invention.

Dans une première étape, les graines, ou une partie spécifique de la plante (feuilles, tubercules, racines, etc.) sont broyées à l'aide d'un broyeur à plantes. La poudre ainsi obtenue peut ultérieurement être "délipidée" à l'aide d'un solvant organique classique (comme par exemple un alcool, l'hexane ou de l'acétone).

Lorsqu'il s'agit de levures, dans une première étape, celles-ci sont mises en culture de manière classique dans un milieu adapté à leur développement, de préférence en présence de lactose. Elles sont récoltées par centrifugation puis mises en suspension dans une solution tampon, préférentiellement un tampon phosphate. Dans une seconde étape, ces cellules sont éclatées à l'aide d'une presse de French ou à l'aide d'un broyeur à bille, la majorité des composants membranaires insolubles étant écartés par centrifugation ou par filtration.

On réalise ensuite l'extraction des protéines suivant le procédé classique (Osborne, 1924) modifié ; le broyat de plante ou le lysat de levures est mis en suspension dans une solution alcaline contenant un produit adsorbant de type polyvinylpolypyrrolidone (PVPP) insoluble (0,01 -20 %) ; en effet il a été observé que les opérations d'hydrolyses et de purifications ultérieures étaient facilitées par ce moyen. En particulier, la concentration en substances de type phénoliques, interagissant avec les protéines, se trouve nettement réduite.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques. On peut alors citer l'utilisation des endoprotéases d'origine végétale (papaïne, bromelaïne, ficine) et de micro-organismes (Aspergillus, Rhizopus, Bacillus, etc.). Les conditions d'hydrolyses sont choisies pour favoriser l'enrichissement en peptide bioactif.

Pour les mêmes raisons que précédemment, c'est-à-dire l'élimination des substances polyphénoliques, une quantité de polyvinylpolypyrrolidone est additionnée au milieu réactionnel lors de cette étape d'hydrolyse ménagée. Après filtration, permettant d'éliminer les enzymes et les polymères, le filtrat (solution) obtenu constitue une première forme du principe actif selon l'invention.

L'hydrolysat obtenu à ce stade peut être encore purifié afin de sélectionner les fractions de bas poids moléculaires, préférentiellement inférieures à 6 kDa, et les peptides générés selon leur nature. Le fractionnement peut s'effectuer avantageusement par des étapes d'ultrafiltrations successives à travers des filtres de porosité décroissante, en conservant les filtrats à chaque étape et/ ou par une méthode de type chromatographique, afin d'enrichir spécifiquement l'hydrolysat en peptide bioactif.

On procède à une phase de dilution dans de l'eau ou dans tout mélange contenant de l'eau, puis stérilisée par ultrafiltration afin d'obtenir un hydrolysat peptidique caractérisé par une teneur en protéines de 0,5 à 5,5 g/l. Cet hydrolysat peptidique correspond à la forme la plus purifiée du principe actif selon l'invention.

L'hydrolysat peptidique obtenu selon l'invention est analysé qualitativement et quantitativement en chromatographie liquide haute pression (CLHP), permettant d'analyser les protéines ayant des poids moléculaires de 0,2 à 25 kDa (selon un gradient de solvants approprié). Les différentes fractions peptidiques qui ont pu être isolées sont ensuite analysées pour leur efficacité biologique. Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif.

L'hydrolysat obtenu est composé de peptides de poids moléculaire inférieur à 6 kDa et enrichi en peptide bioactif de 4 à 6 acides aminés comprenant au moins un résidu glycine, un résidu leucine et un résidu acide glutamique.

Le deuxième objet de la présente invention est une composition comprenant, dans un milieu physiologiquement acceptable, en tant que principe actif capable de renforcer la fonction barrière de l'épiderme, l'hydrolysat peptidique enrichi en peptide bioactif selon l'invention à une concentration comprise entre 0,0001 % et 20 % environ, et préférentiellement à une concentration comprise entre 0,05 % et 5 % environ par rapport au poids total de la composition finale.

Selon un mode de réalisation avantageux de l'invention, le principe actif selon l'invention est solubilisé dans un ou plusieurs solvants physiologiquement acceptables, classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, le principe actif selon l'invention est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur physiologiquement acceptable.

La composition utilisable selon l'invention peut en particulier consister en une composition pour soins capillaires, et notamment un shampooing, un après-shampooing, une lotion traitante, une crème ou un gel coiffant, une lotion restructurante pour les cheveux, un masque, etc. La composition peut également se présenter sous forme de teinture ou dé mascara à appliquer au pinceau ou au peigne, en particulier sur les cils, les sourcils ou les cheveux.

La composition utilisable selon l'invention pourra être appliquée par toute voie appropriée, notamment orale, parentérale ou topique, et la formulation des compositions sera adaptée par l'homme du métier, en particulier pour des compositions cosmétiques ou dermatologiques. Avantageusement, les compositions selon l'invention sont destinées à une administration par voie topique. Ces compositions doivent donc contenir un milieu physiologiquement acceptable, c'est-à-dire compatible avec la peau et les phanères, et couvrent toutes les formes cosmétiques ou dermatologiques.

Il est bien entendu que le principe actif selon l'invention peut être utilisé seul ou bien en association avec d'autres principes actifs.

Avantageusement, les compositions utilisables selon l'invention contiennent, en outre, divers principes actifs protecteurs ou antivieillissement, destinés à favoriser et à compléter l'action dudit principe actif. On peut citer, de marnière, non limitative, les ingrédients suivants : des agents cicatrisants, anti-âge, antirides, apaisants, anti-radicalaires, anti-UV, des agents stimulant la synthèse de macromolécules dermiques ou le métabolisme énergétique, des agents hydratants, antibactériens, antifongiques, anti-inflammatoires, anesthésiques, dés agents modulants la différenciation, la pigmentation ou la dépigmentation cutanée, des agents stimulant la pousse des ongles ou des cheveux. Préférentiellement, on utilisera un agent présentant une activité antiride, tel qu'un agent anti-radicalaire ou antioxydant, ou un agent stimulant la synthèse de macromolécules dermiques, agent stimulant le métabolisme énergétique, inhibiteur de métallo-protéinase.

Par exemple, il peut être ajouté d'autres principes actifs ayant une action anti-radicalaire ou antioxydante, choisis parmi la vitamine C, la vitamine E, le coenzyme Q10 et les extraits polyphénoliques de plantes, les rétinoïdes.

La composition selon l'invention peut encore associer au principe actif selon l'invention, d'autres principes actifs stimulant les synthèses des macromolécules dermiques (laminine, fibronectine, collagène), par exemple le peptide de collagène commercialisé sous le nom « Collaxyl^{®} » par la société Vincience.

La composition selon l'invention peut également associer au principe actif selon l'invention, d'autres principes actifs stimulant le métabolisme énergétique, comme le principe actif commercialisé sous la dénomination « GP4G^{®} » par la société Vincience.

Il est bien évident que l'invention s'adresse aux mammifères en général, et plus particulièrement aux êtres humains.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

L'ensemble de ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, d'autres principes actifs cosmétiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à une concentration allant de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

L'invention a pour troisième objet une composition pharmaceutique comprenant une quantité efficace d'hydrolysat peptidique selon l'invention, à titre de médicament. Par exemple la composition pharmaceutique pourra être destinée à prévenir ou traiter les pathologies caractérisées par une altération de la fonction barrière, telles que les peaux hypersensibles, irritées, ou réactives et l'eczéma atopique.

Selon cette forme de l'invention, les compositions seront appropriées à une administration orale pour un usage pharmaceutique. Ainsi les compositions pourront notamment se présenter sous forme de comprimés, capsules, gélules, pâtes à mâcher, poudres à consommer telles quelles ou à mélanger extemporanément avec un liquide, sirop, gels, et toute autre forme connue de l'homme du métier. Ces compositions comprennent, en outre, tout additif communément utilisé dans le domaine d'application envisagé ainsi que les adjuvants nécessaires à leur formulation, tels que des solvants, des épaississants, des diluants, des antioxydants, des conservateurs, d'autres principes actifs pharmaceutiques, des huiles essentielles, des vitamines, des acides gras essentiels, etc.

L'invention a pour quatrième objet l'utilisation cosmétique, dans une composition, d'une quantité efficace d'hydrolysat peptidique selon l'invention, en tant que principe actif activateur de la HMG-CoA réductase humaine, pour renforcer la fonction barrière de l'épiderme et stimuler la différenciation épidermique.

La quantité efficace de principe actif correspond à la quantité nécessaire pour obtenir le résultat recherché, à savoir, activer la HMG-CoA réductase, dans le but d'améliorer la fonction barrière de l'épiderme et de stimuler la différenciation épidermique.

On entend par « renforcer la fonction barrière cutanée et stimuler la différenciation épidermique », l'amélioration de la capacité de protection de la couche cornée et l'augmentation de l'expression de marqueurs biologiques de différenciations, tels que les kératines..

Ainsi, ledit principe actif, grâce à ses propriétés particulières, pourra être utilisé dans une composition cosmétique destinée à renforcer la fonction barrière cutanée et à stimuler la différenciation épidermique.

D'autre part, l'hydrolysat peptidique pourra être utilisé avantageusement, en tant que principe actif, dans une composition cosmétique destinée à lutter de manière préventive et/ou curative contre les signes du vieillissement cutané, et plus particulièrement du vieillissement cutané photo-induit (photo-vieillissement). Par signes cutanées du vieillissement ou du photo-vieillissement, on entend toutes modifications de l'aspect extérieur de la peau et des phanères dues au vieillissement comme, par exemple, les rugosités superficielles de la couche cornée, les rides et ridules, mais également toute modification interne de la peau qui ne se traduit pas systématiquement par un aspect extérieur modifié comme, par exemple, l'amincissement de l'épiderme ou toute autre dégradation interne de la peau consécutive à une exposition aux rayonnements ultraviolets (UV).

Selon un autre aspect de l'invention, l'hydrolysat peptidique pourra être utilisé avantageusement, en tant que principe actif, dans une composition cosmétique destinée à protéger la peau contre tous types d'agressions extérieures.

On entend par l'expression « agression extérieure », lès agressions que peut produire l'environnement. A titre d'exemple, on peut citer des agressions telles que la pollution, les UV, ou encore les produits à caractère irritant tels que les tensioactifs, les conservateurs ou les parfums, les agressions mécaniques, telles que les abrasions, le rasage ou l'épilation. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette. La sècheresse de l'atmosphère est également une cause importante d'agression cutanée. Ces agressions extérieures aboutissent à une altération de la fonction barrière qui se traduit par un inconfort cutané, des phénomènes sensoriels désagréables, tels que des tiraillements ou des démangeaisons, voire des fragilités excessives et des rougeurs.

En particulier, l'invention a pour objet l'utilisation cosmétique de l'hydrolysat selon l'invention dans une composition destinée à prévenir ou traiter les dommages causés à la peau par des agressions extérieures de la peau, choisies parmi les traitements mécaniques tels que le rasage ou l'épilation, les lessivages trop intenses par les détergents, les conditions climatiques extrêmes ou les variations brutales de température et d'hygrométrie.

L'invention a pour cinquième objet un procédé de traitement cosmétique destiné à prévenir et/ou à lutter contre les signes cutanés du vieillissement et/ou du photo-vieillissement, selon lequel on applique une composition comprenant une quantité efficace d'hydrolysat peptidique selon l'invention sur les zones à traiter.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

### Liste des figures

Figure 1 : Exemple de chromatogramme obtenu par CLHP, avec mise en évidence du pic correspondant au peptide bioactif dans un hydrolysat d'épeautre
Figure 2 : Exemple de chromatogramme obtenu par CLHP, avec mise en évidence du pic correspondant au peptide bioactif dans un hydrolysat de pomme de terre
Figure 3 : Exemple de chromatogramme obtenu par CLHP, avec mise en évidence du pic correspondant au peptide bioactif dans un hydrolysat de maïs
Figure 4 : Exemple de chromatogramme obtenu par CLHP, avec mise en évidence du pic correspondant au peptide bioactif dans un hydrolysat de pois
Figure 5 : Exemple de chromatogramme obtenu par CLHP, avec mise en évidence du pic correspondant au peptide bioactif dans un hydrolysat de soja
Figure 6 : Exemple de chromatogramme obtenu par CLHP, avec mise en évidence du pic correspondant au peptide bioactif dans un hydrolysat de Saccharomyces cerevisiae

### Exemple 1 : Préparation d'un hydrolysat peptidique à partir d'épeautre (Triticum monococcum)

Les grains d'épeautre (*Triticum monococcum*) sont mis en solution dans 10 volumes d'eau en présence de 2 % de POLYCLAR® 10 (polyvinylpyrrolidone - PVPP - insoluble). Le mélange est ajusté à un pH compris entre 6 et 8 avec une solution aqueuse de soude 1 M.

Après ajustement du pH, une amylase (hasidase®) et une protéase (papaïne à 2 %) sont ajoutées dans le milieu réactionnel. L'hydrolyse est obtenue après 2 heures d'agitation à 50°C. On procède ensuite à l'inactivation de l'enzyme par chauffage de la solution à 80°C pendant 2 heures. Après centrifugation, la solution aqueuse surnageante correspondant à un hydrolysat brut d'épeautre est récupérée. Les conditions d'hydrolyse ont été choisies de façon à permettre un enrichissement en peptide bioactif de 4 à 6 acides aminés contenant les résidus Gly, Leu et Acide glutamique.

Le procédé de purification de l'hydrolysat brut commence par des filtrations successives à l'aide de filtres-plaques Seitz-Orion de porosité décroissante (jusqu'à 0,2 µm) afin d'obtenir une solution brillante et limpide, qualifiée d'hydrolysat 1.

A cette étape, l'hydrolysat 1 d'épeautre est caractérisé par une couleur jaune clair et par un extrait sec titrant de 20 à 25 g/kg, un taux de protéines de 10 à 12 g/l et un taux de sucres de 5 à 8 g/l.

La nature protéique de l'hydrolysat 1 est mise en évidence après analyse par électrophorèse sur gel de polyacrylamide NuPAGE® Bis-Tris Pre-cast (Invitrogen). L'hydrolysat protéique d'épeautre est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites ne se ré-oxydent durant l'électrophorèse. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe une bande à 24 kDa, correspondant à l'enzyme, puis des protéines inférieures à 6 kDa.

L'hydrolysat 1 est ensuite purifié par ultrafiltration à l'aide de la cassette Pellicon® 2 Biomax 5 kDa afin d'éliminer toutes traces d'enzymes. En fin de purification, on obtient un hydrolysat peptidique jaune-orangé, brillant et limpide. On procède à une phase de dilution afin d'obtenir un hydrolysat peptidique caractérisé par une teneur en protéines de 1,5 à 3,5 g/l. Cet hydrolysat peptidique correspond au principe actif selon l'invention. Cet hydrolysat peptidique est alors analysé en chromatographie liquide haute pression (CLHP) à l'aide d'un appareil HP1100 piloté par le logiciel ChemStation. La colonne utilisée lors de l'élution de l'hydrolysat est une Nucleosil® 300-5 C4 MPN (125 x 4 mn), permettant de chromatographier des protéines ayant des poids moléculaires de 0.2 à 25 kDa dans les conditions suivantes :
Un gradient Méthanol:
   - Colonne Uptisphere OPB 125 x 3 mm
   - Solvant A: eau grade HPLC contenant 0,1 % d'acide heptafluorobutyrique (HFBA)
   - Solvant B: méthanol grade HPLC
   - Gradient: 100 % à 40 % solvant A en 13 min puis de 40 % à 10 % en 5 min

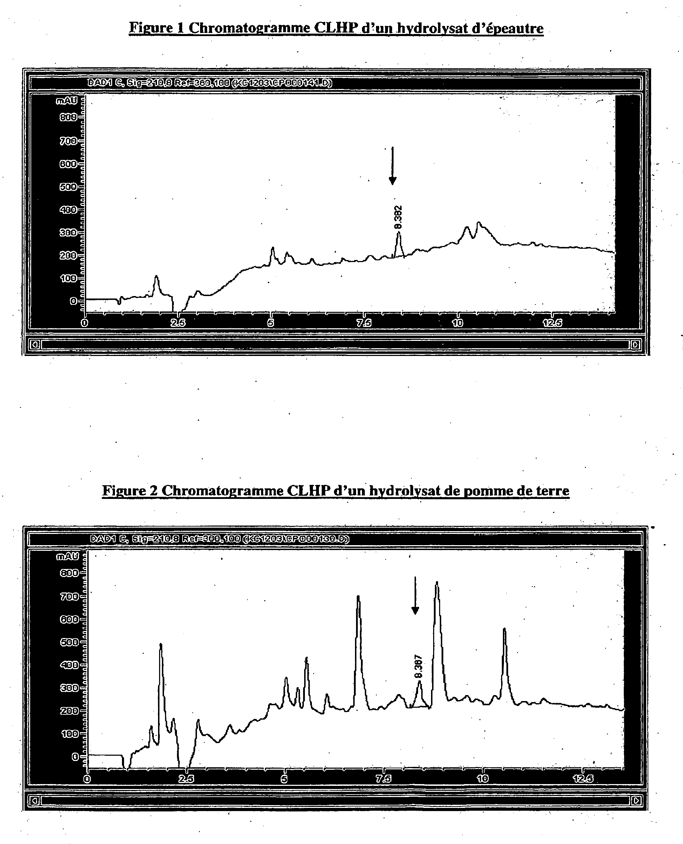

Dans ces conditions chromatographiques, plusieurs fractions peptidiques ont pu être isolées. Un exemple de chromatogramme obtenu par CLHP (chromatographie en phase liquide à haute pression), avec mise en évidence du pic correspondant au peptide bioactif est donné à la figure 1.

Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

Un exemple de composition en acides aminés de l'hydrolysat est donné dans le tableau suivant (en %) :

| Acides aminés | % |
|---|---|
| Alanine | 3,1 |
| Acide Aspartique | 4,7 |
| Arginine | 3,1 |
| Acide Glutamique | 32,8 |
| Glycine | 3,1 |
| Histidine | < 3,0 |
| Isoleucine | < 5,5 |
| Leucine | 6,2 |
| Lysine | < 2,2 |
| Phénylalanine | 4,5 |
| Proline | 10,9 |
| Seriné | 4,7 |
| Thréonine | 3,1 |
| Tyrosine | < 3,6 |
| valine | 4,7 |
| Tryptophane | < 1,5 |

### Exemple 2 Préparation d'un hydrolysat peptidique à partir de tubercules appartenant à l'espèce Solanum tuberosum

Les tubercules de pomme de terre (*Solanum tuberosum*) sont mis en solution dans 10 volumes d'eau en présence de 2 % de POLYCLAR® 10 (polyvinylpyrrolidone - PVPP - insoluble). Le mélange est ajusté à un pH compris entre 6 et 8 avec une solution aqueuse de soude 1 M. Une précipitation en milieu acide est ensuite réalisée. Le culot est remis en solution et après ajustement du pH, de la papaïne à 2 % est ajoutée dans le milieu réactionnel. L'hydrolyse est obtenue après 2 heures d'agitation à 55°C. On procède ensuite à l'inactivation de l'enzyme par chauffage de la solution à 80°C pendant 2 heures. Après centrifugation, la solution aqueuse surnageante correspondant à un hydrolysat brut de pomme de terre est récupérée. Les conditions d'hydrolyse ont été choisies de façon à permettre un enrichissement en peptide bioactif de 4 à 6 acides aminés contenant les résidus Gly, Leu et Glu.

Le procédé de purification de l'hydrolysat brut commence par des filtrations successives à l'aide de filtres-plaques Seitz-Orion de porosité décroissante (jusqu'à 0,2 µm) afin d'obtenir une solution jaune brillante et limpide, qualifié d'hydrolysat 1.

A cette étape, l'hydrolysat 1 de pomme de terre est caractérisé par un extrait sec titrant de 40 à 60 g/kg, un taux de protéines de 20 à 25 g/l et un taux de sucres de 1 à 3 g/l.

La nature protéique de l'hydrolysat 1 est mise en évidence après analyse par électrophorèse sur gel de polyacrylamide NUAGE® Bis-Tris Pre-cast (Invitrogen). L'hydrolysat protéique de pomme de terre est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites ne se ré-oxydent durant l'électrophorèse. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe que les protéines obtenues ont un poids moléculaire inférieur à 6 kDa.

L'hydrolysat 1 est ensuite purifié afin de ne conserver que les peptides de poids moléculaire inférieur à 5 kDa, à l'aide d'une filtration à flux tangentiel. Pour cela, l'hydrolysat 1 est pompé sous pression à travers un support Pellicon® équipé de cassette Pellicon® 2 Biomax 5 kDa. En fin de purification, on obtient un hydrolysat peptidique brillant et limpide. On procède ensuite à une phase de dilution afin d'obtenir un hydrolysat peptidique caractérisé par une teneur en protéines de 3,5 à 5,5 g/l. Cet hydrolysat peptidique correspond au principe actif selon l'invention.
Cet hydrolysat peptidique est alors analysé en chromatographie liquide haute pression (CLHP) à l'aide d'un appareil HP1100 piloté par le logiciel ChemStation. La colonne utilisée lors de l'élution de l'hydrolysat est une Nucleosil® 300-5 C4 MPN (125 x 4 mn), permettant de chromatographier des protéines ayant des poids moléculaires de 0.2 à 25 kDa (selon des conditions identiques à l'exemple 1). Dans ces conditions chromatographiques, plusieurs fractions peptidiques ont pu être isolées.

Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif.

Un exemple de chromatogramme obtenu par CLHP (chromatographie en phase liquide à haute pression), avec mise en évidence du pic correspondant au peptide bioactif est donné à la figure 2.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

Un exemple de composition en acides aminés de l'hydrolysat est donné dans le tableau suivant (en %) :

| Acides aminés | % |
|---|---|
| Alanine | 7,8 |
| Acide Aspartique | 20,1 |
| Arqinine | 7,3 |
| Acide Glutamique | 17,4 |
| Glycine | 7,3 |
| Histidine | 3,2 |
| Isoleucine | 9,1 |
| Leucine | 15,1 |
| Lysine | 11,4 |
| Phénylalanine | 8,7 |
| Proline | 7,7 |
| Serine | 8,7 |
| Thréonine | 9,1 |
| Tyrosine | 8,2 |
| valine | 10,5 |
| Tryptophane | 1,4 |

### Exemple 3 : Préparation d'un hydrolysat peptidique à partir de tourteaux de maïs (Zea mays L.)

Le tourteau de maïs (*Zea mays L.*) est mis en solution dans 10 volumes d'eau en présence de 2 % de POLYCLAR® 10 (polyvinylpyrrolidone - PVPP - insoluble). Le mélange est ajusté à un pH compris entre 6 et 8 avec une solution aqueuse de soude 1 M.

Après ajustement du pH, de la papaïne à 2 % est ajoutée dans le milieu réactionnel. L'hydrolyse est obtenue après 2 heures d'agitation à 55°C. On procède ensuite à l'inactivation de l'enzyme par chauffage de la solution à 80°C pendant 2 heures. Après centrifugation, la solution aqueuse surnageante correspondant à un hydrolysat brut de maïs est récupérée. Les conditions d'hydrolyse ont été choisies de façon à permettre un enrichissement en peptide bioactif de 4 à 6 acides aminés contenant les résidus Gly, Leu et Glu.

Le procédé de purification de l'hydrolysat brut commence par des filtrations successives à l'aide de filtres-plaques Seitz-Orion de porosité décroissante (jusqu'à 0,2 µm) afin d'obtenir une solution jaune, brillante et limpide, qualifié d'hydrolysat 1.

A cette étape, l'hydrolysat 1 de maïs est caractérisé par un extrait sec titrant de 20 à 30 g/kg, un taux de protéines de 20 à 25 g/l et un taux de sucres de 2 à 5 g/l.

La nature protéique de l'hydrolysat 1 est mise en évidence après analyse par électrophorèse sur gel de polyacrylamide NuPAGE® Bis-Tris Pre-cast (Invitrogen). L'hydrolysat protéique de maïs est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites ne se ré-oxydent durant l'électrophorèse. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe des protéines de poids moléculaire inférieur à 6 kDa.

L'hydrolysat 1 est ensuite purifié en éliminant les protéines de haut poids moléculaire par ultrafiltration à l'aide de la cassette Pellicon® 2 Biomax 5 kDa afin de ne conserver que les composés de nature peptidique inférieurs à 5 kDa,

Après cette purification finale, on procède à une phase de dilution afin d'obtenir un hydrolysat peptidique caractérisé par un taux de protéines compris entre 3,5 et 5,5 g/l. Cet hydrolysat peptidique correspond au principe actif selon l'invention.

Cet hydrolysat peptidique est alors analysé en chromatographie liquide haute pression (CLHP) à l'aide d'un appareil HP1100 piloté par le logiciel ChemStation. La colonne utilisée lors de l'élution de l'hydrolysat est une Nucleosil® 300-5 C4 MPN (125 x 4 mn), permettant dechromatographier des protéines ayant des poids moléculaires de 0.2 à 25 kDa (selon un gradient de solvants approprié, identique à l'exemple 1). Dans ces conditions chromatographiques, plusieurs fractions peptidiques ont pu être isolées.

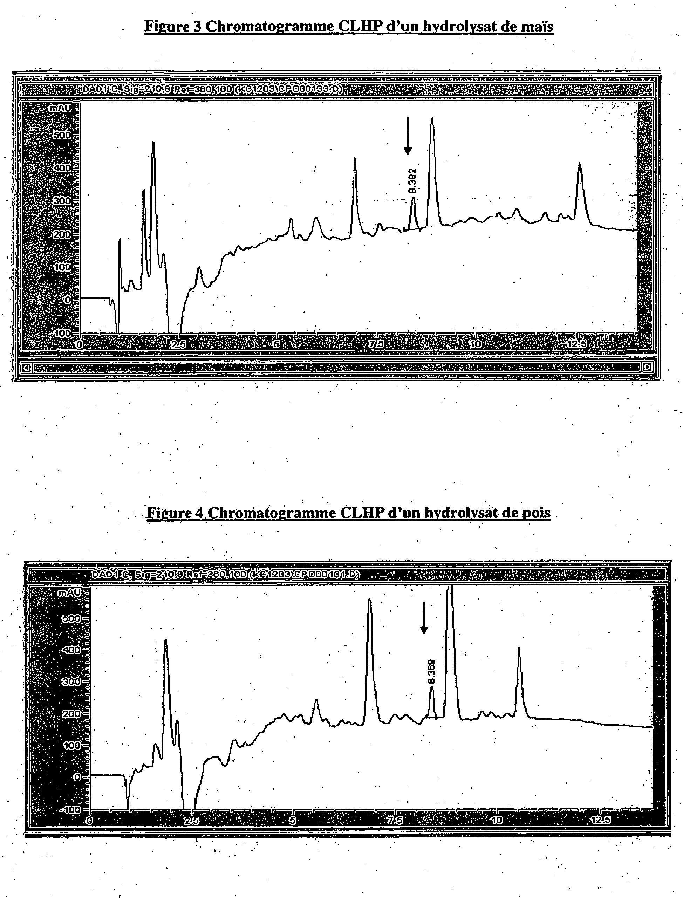

Un exemple de chromatogramme obtenu par CLHP (chromatographie en phase liquide à haute pression), avec mise en évidence du pic correspondant au peptide bioactif est donné à la figure 3

Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate). Un exemple de composition en acides aminés de l'hydrolysat est donné dans le tableau suivant (%) :

| Acides aminés | % |
|---|---|
| Alanine | 9,4 |
| Acide Aspartique | 7,2 |
| Arginine | 3,6 |
| Acide Glutamique | 23,7 |
| Glycine | 3,6 |
| Histidine | 2,2 |
| Isoleucine | 4,5 |
| Leucine | 16,1 |
| Lysine | 2,2 |
| Phénylalanine | 6,7 |
| Proline | 10,3 |
| Serine | 6,3 |
| Thréonine | 4,0 |
| Tyrosine | 5,8 |
| valine | 5,4 |
| Tryptophane | < 0,5 |

### Exemple 4: Préparation d'un hydrolysat peptidique à partir de pois (Pisum sativum L.)

L'hydrolysat peptidique est obtenu à partir d'un extrait de plantes de l'espèce *Pisum sativum L.* Bien entendu l'extrait peut être préparé à partir de plantes d'au moins l'une quelconque des nombreuses variétés et espèces appartenant au genre Pisum.

Dans une première étape, 1 kg de pois décortiqués sont délipidés par l'action d'un solvant organique : l'hexane.

La farine de pois ainsi obtenue est mise en solution dans 10 volumes d'eau en présence de 2 % de POLYCLAR® 10 (polyvinylpyrrolidone - PVPP - insoluble). Le mélange est ajusté à un pH compris entre 6 et 7 avec une solution aqueuse de soude 1 M.

Après ajustement du pH, il est rajouté 2 % de flavourzym® dans le milieu réactionnel. L'hydrolyse est obtenue après 2 heures d'agitation à 50°C. On procède à l'inactivation de l'enzyme en chauffant la solution à 80°C pendant 2 heures. Le mélange réactionnel ainsi obtenu correspond à l'extrait de pois. Les conditions d'hydrolyse ont été choisies de façon à permettre un enrichissement en peptide bioactif de 4 à 6 acides aminés contenant les résidus Gly, Leu et Acide glutamique.

Le procédé de purification commence par des filtrations successives à l'aide de filtres-plaques Seitz-Orion de porosité décroissante (jusqu'à 0,2 µm) afin d'obtenir une solution brillante et limpide. A cette étape, l'hydrolysat de pois est caractérisé par un sec de 70-80 g/kg, un taux de protéines de 55-65 g/l, un taux de sucres de 2-5 g/l et un taux de polyphénol de 1-3 g/l.

La nature protéique de cet hydrolysat est mise en évidence par électrophorèse sur gel de polyacrylamide. Pour cette analyse, on utilise les gels NuPAGE® Bis-Tris Pre-cast (Invitrogen). L'hydrolysat peptidique de pois est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites se ré-oxydent durant l'électrophorèse. La migration des protéines est réalisée à l'aide du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe 2 grandes familles de protéines : la 1 ère famille correspond à des protéines de poids moléculaire de 25 à 20 kDa et la dernière famille à des protéines de poids moléculaires inférieurs à 5kDa.

Cette solution est alors purifiée en éliminant les protéines de poids moléculaires supérieurs à 5 kDa à l'aide de filtration à flux tangentiel.

Pour cela, l'hydrolysat de pois est pompé sous pression à travers un support Pellicon® équipé de cassette Pellicon® 2 Biomax 30 kDa. Ce 1er filtrat est récupéré pour être ensuite filtré à travers une autre cassette Pellicon® 2 Biomax 5 kDa. En fin de purification, on obtient un hydrolysat peptidique de pois jaune-beige, brillant et limpide. Il est caractérisé par un sec de 50 à 55 g/kg, une teneur en protéines de 50 à 52 g/l.

Cette solution est alors analysée en chromatographie liquide haute pression à l'aide d'un appareil HP1100 piloté par le logiciel ChemStation. La colonne utilisée lors de l'élution de l'extrait de pois est une Nucleosil® 300-5 C4 MPN (125 x 4 mn). Cette colonne permet de chromatographier des protéines ayant des poids moléculaires de 0,2 à 25 kDa (selon un gradient de solvants approprié, identique à l'exemple 1). Dans ces conditions chromatographiques, plusieurs fractions peptidiques ont pu être isolées.

Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif. Un exemple de chromatogramme obtenu par CLHP (chromatographie en phase liquide à haute pression), avec mise en évidence du pic correspondant au peptide bioactif est donné à la figure 4.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

### Exemple 5 : Préparation d'un hydrolysat peptidique à partir de tourteau de soja (Glycine Max. L.)

Le principe actif est obtenu à partir de plantes de l'espèce *Glycine max L.* Bien entendu l'extrait peut être préparé à partir de plantes d'au moins l'une quelconque des nombreuses variétés et espèces appartenant au genre Glycine. Les tourteaux sont les résidus solides obtenus après extraction de l'huile des graines de soja. Ils représentent de 50 à 75 % de la masse des graines.

Dans une première étape, 1 kg de tourteau de soja est broyé dans un broyeur à céréales. La farine obtenue est délipidée par l'action d'un solvant organique, l'hexane. Après filtration et séchage sous vide, la poudre obtenue est mise en suspension dans une solution aqueuse alcaline (dilution au 1/10) pH 10, contenant 1 % de polyvinylpolypyrrolidone (Polyclar V ISP). Ce mélange est maintenu sous agitation pendant un temps suffisamment long pour permettre la solubilisation des fractions solubles. La température d'extraction est variable (comprise entre 4 et 80°C) ; préférentiellement l'opération sera réalisée à froid. Après cette phase d'extraction, le milieu est clarifié par centrifugation puis filtré sur filtre à plaque. Ce filtrat qui contient les fractions solubles du soja est ensuite soumis à une précipitation des protéines en faisant varier la force ionique en milieu neutre ou acide, ce qui permet d'éliminer les composants glucidiques solubles, les lipides et les acides nucléiques. Le milieu est amené à pH 3,5. Le surnageant est éliminé et le précipité est ensuite lavé à l'aide d'un solvant tel que, par exemple, l'éthanol ou le méthanol, puis le solvant est évaporé par séchage sous vide.

A ce stade, on obtient environ 50 grammes de poudre de couleur jaune clair d'extrait protéique brut contenant :
- Protéines : 75 %
- Glucides : 20 %
- Lipides : 5 %

Le précipité riche en protéines est remis en solution dans l'eau ou un autre solvant.

L'extrait protéique brut est alors soumis à une série d'hydrolyses ménagées et sélectives consistant en des hydrolyses chimiques et enzymatiques en présence de 0,5 % de PVPP (Polyclar V) et d'endopeptidases à cystéine (papaïne, ficine). Après réaction l'hydrolysat est filtré sur plaque puis sur cartouche stérilisante (0,2 µm).

On obtient alors un hydrolysat de couleur claire, titrant de 15 à 30 g/l d'extrait sec, qui est alors dilué de telle sorte que la concentration en composés de nature peptidique déterminée, par la méthode de Lowry, soit comprise entre 0,1 et 5g/l et préférentiellement entre 0,5 et 2 g/l. L'analyse physico-chimique de l'hydrolysat peptidique, qui constitue le principe actif, montre que son pH est compris entre 4 et 7, et préférentiellement entre 5 et 6, l'extrait sec titre de 1 à 8 g/l et de manière préférée de 2 à 5 g/l, sa teneur en composés de nature peptidique est comprise entre 0,1 et 5g/l, et préférentiellement, entre 0,5 et 2 g/l et sa teneur en sucres entre 0,5 à 2,5 g/l. Les conditions d'hydrolyse ont été choisies de façon à permettre un enrichissement en peptide bioactif de 4 à 6 acides aminés contenant les résidus Gly, Leu et Acide glutamique.

On procède ensuite à une ultrafiltration de la solution sur une cartouche de filtration Millipore Helicon (seuil de coupure 1 kDa). Les hauts poids moléculaires contenus dans le retentât sont écartés, le filtrat est conservé.

Cette solution est alors analysée en chromatographie liquide haute pression à l'aide d'un appareil HP1100 piloté par le logiciel ChemStation. La colonne utilisée lors de l'élution de l'hydrolysat de soja est une Nucleosil® 300-5 C4 MPN (125 x 4 mn). Cette colonne permet de chromatographier des protéines ayant des poids moléculaires de 0,2 à 25 kDa (selon un gradient de solvants approprié, identique à l'exemple 1). Dans ces conditions chromatographiques, plusieurs fractions peptidiques ont été isolées.

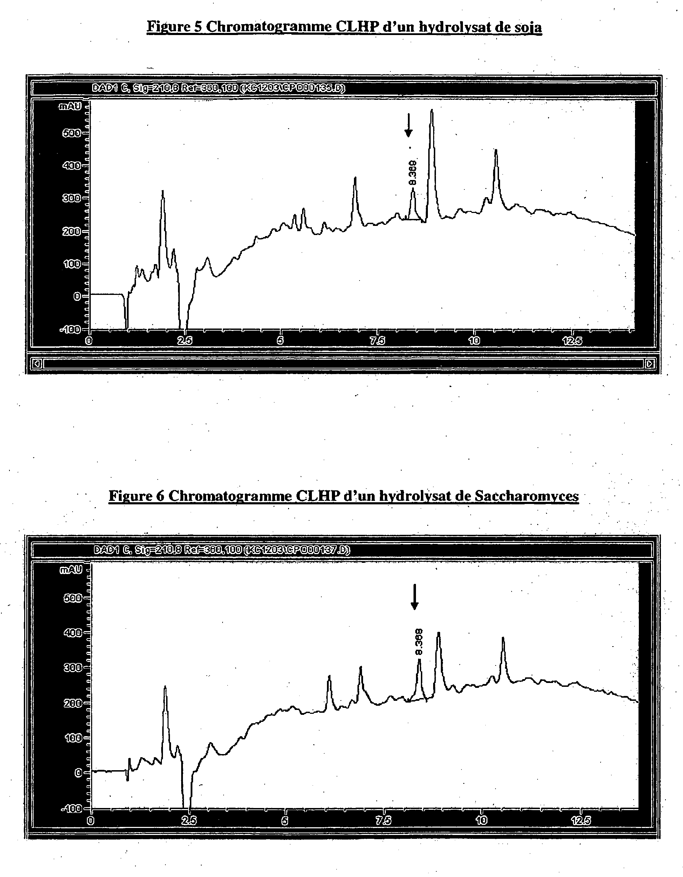

Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif. Un exemple de chromatogramme obtenu par CLHP (chromatographie en phase liquide à haute pression), avec mise en évidence du pic correspondant au peptide bioactif, est donné à la figure 5.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

Une variante du protocole consiste à effectuer une purification du principe actif, obtenu selon le protocole précédent, par chromatographie d'échange d'ions, sur une colonne TSK gel (TosoHaas) avec un tampon phosphate pH 7, afin d'enrichir l'hydrolysat peptidique en peptide bioactif.

### Exemple 6 : Préparation d'un hydrolysat peptidique à partir de levures Saccharomyces cerevisiae

L'hydrolysat peptidique peut être obtenu à partir d'un extrait de levures de l'espèce *Saccharomyces cerevisiae.* Les levures sont cultivées dans un milieu adapté à leur développement, de préférence en présence de lactose, puis centrifugées pour récupérer une biomasse. La biomasse de saccharomyces est mise en solution dans 10 volumes d'eau en présence de 2 % de POLYCLAR® 10 (polyvinylpyrrolidone - PVPP - insoluble) et 0,2 % de charbon actif. Le mélange est ajusté à un pH compris entre 6 et 7,5 avec une solution aqueuse de soude 1 M.

Après ajustement du pH, il est rajouté 2 % de papaïne dans le milieu réactionnel. L'hydrolyse est obtenue après 2 heures d'agitation à 55°C. On procède à l'inactivation de l'enzyme en chauffant la solution à 80°C pendant 2 heures. Après centrifugation, le mélange réactionnel correspondant à l'extrait de saccharomyces est alors obtenu. Les conditions d'hydrolyse ont été choisies de façon à permettre un enrichissement en peptide bioactif de 4 à 6 acides aminés contenant les résidus Gly, Leu et Acide glutamique.

Le procédé de purification commence par des filtrations successives à l'aide de filtres-plaques Seitz-Orion de porosité décroissante (jusqu'à 0,2 µm) afin d'obtenir une solution brillante et limpide. A cette étape, l'extrait de saccharomyces est caractérisé par un sec de 25 à 35 g/kg, un taux de protéines de 10 à 15 g/l et un taux de sucres de 5-10 g/l.

La nature protéique de cet extrait est mise en évidence par électrophorèse sur gel de polyacrylamide. Pour cette analyse, les gels NuPAGE® Bis-Tris Pre-cast (Invitrogen) sont utilisés. L'hydrolysat peptidique est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites se ré-oxydent durant l'électrophorèse. La migration des protéines est réalisée à l'aide du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe 3 grandes familles de protéines : la 1ère famille correspond à des protéines de poids moléculaire supérieur à 75 kDa, la 2ème famille à des protéines de 20 à 25 kDa et la dernière famille à des protéines de poids moléculaire inférieur à 5kDa.

Cette solution est alors purifiée en éliminant les protéines de poids moléculaire supérieur à 5 kDa à l'aide de filtration à flux tangentiel.

Pour cela, l'hydrolysat peptidique de saccharomyces est pompé sous pression à travers un support Pellicon®, équipé de cassette Pellicon® 2 Biomax 50 kDa. Ce 1er filtrat est récupéré pour être ensuite filtré à travers une seconde cassette Pellicon® 2 Biomax 10 kDa. Il est alors récupéré un second filtrat qui est encore élué à travers une dernière cassette Pellicon® 2 Biomax 5 kDa. En fin de purification, on obtient un extrait végétal de saccharomyces beige, brillant et limpide. Il est caractérisé par un sec de 35 à 45 g/kg, une teneur en protéines de 30 à 40 g/l.

Cette solution est alors analysée en chromatographie liquide haute pression à l'aide d'un appareil HP1100 piloté par le logiciel ChemStation. La colonne utilisée lors de l'élution de l'hydrolysat de saccharomyces est une Nucleosil® 300-5 C4 MPN (125 x 4 mn). Cette colonne permet de chromatographier des protéines ayant des poids moléculaires de 0,2 à 25 kDa (selon un gradient de solvants approprié, identique à l'exemple 1) Dans ces conditions chromatographiques, il a été isolé plusieurs fractions peptidiques.

Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

### Exemple 7 : Etude ultrastructurale des corps lamellaires dans des kératinocytes humains traités par l'hydrolysat selon l'exemple 1

Le but de cette étude est d'étudier de manière ultrastructurale, en microscopie électronique à transmission, des kératinocytes traités par l'hydrolysat selon l'exemple 1 à 1%.

Protocole : Des kératinocytes humains normaux en culture sont traités avec une solution à 1 % d'hydrolysat selon l'exemple 1 pendant 48 heures (le milieu en présence de l'actif est changé tous les 24 heures). Les cellules sont lavées au PBS, puis sont fixées par la fixation hypertonique de Karnosky (4 % Paraformaldéhyde, 5 % glutaraldéhyde dans un tampon 0,08M Phosphate) 1 heure à température ambiante puis 24 heures à 4°C. Les cellules sont détachées du support par grattage, centrifugées 5 minutes à 1000 rpm. Le surnageant est éliminé et un tampon de 1 M sodium cacodylate est déposé sur le culot. Les cellules sont mélangées à 2 % d'agar puis post-fixées par le tétroxyde d'osmium 1 heure. Les spécimens sont alors déshydratés par passages successifs dans une série d'alcool (de 50 à 100 %). Les cellules sont ensuite enrobées dans une résine. La polymérisation s'effectue pendant environ 12 heures à 60°C. Des coupes semi fines de 0,5 µm sont réalisées à l'ultra-microtome. Les coupes sont déposées sur une lame collée à la chaleur puis colorée au bleu de toluidine. Les lames sont ensuite déshydratées de nouveau et montées dans un milieu adéquat. Après avoir choisi la zone d'étude optimale, le bloc est retaillé à la taille désirée et des coupes ultrafines sont alors réalisées, seules les coupes qui ont une couleur « gris-argent » et une taille adéquate sont montées sur la grille de microscopie électronique doublement marquée par de l'uranyl acétate et du citrate de plomb, et sont examinées au microscope à transmission à 60 ou 80 KV.

Résultats : L'étude ultrastructurale montre que l'appareil de Golgi est sensiblement plus développé que dans les cellules témoins. Cette augmentation est liée à une surproduction des corps lamellaires (ou corps d'Odland) qui est le signe d'une augmentation de la synthèse lipidique.

Conclusions : L'hydrolysat selon l'exemple 1 à 1 % est capable d'induire une augmentation de la synthèse lipidique dans les kératinocytes humains normaux.

### Exemple 8 : Etude ultrastructurale des « caveolae » dans des fibroblastes humains traités par l'hydrolysat selon l'exemple 2

Le but de cette étude est d'étudier au niveau ultrastructural les cavéoles dans les fibroblastes dermiques humains.

Protocole : Des fibroblastes dermiques humains normaux en culture sont traités avec l'hydrolysat selon l'exemple 2 à 1 % pendant 48 heures (le milieu contenant l'actif est changé tous les 24 heures).

Résultats : l'étude ultrastructurale montre une augmentation importante des cavéoles dans les cellules traitées par l'hydrolysat selon l'exemple 2 à 1 %, en comparaison des cellules témoins non traitées. Ces résultats sont le signe d'un effet positif du principe actif, puisque les cavéoles sont des invaginations de la membrane plasmique qui permettent l'externalisation de molécules telles que le cholestérol.

Conclusion : L'hydrolysat selon l'exemple 2 à 1 % provoque l'augmentation des structures membranaires d'externalisation du cholestérol.

### Exemple 9 : Etude de la différenciation des kératinocytes humains traités par l'hydrolysat selon l'exemple 2

Le but de cette étude est de déterminer l'influence de l'hydrolysat selon l'exemple sur la différenciation épidermique, et plus particulièrement sur l'expression de l'ensemble des cytokératines (ou pankératines), qui sont des marqueurs de la différenciation kératinocytaire.

Protocole : Des kératinocytes humains normaux en culture sont traités avec l'hydrolysat selon l'exemple 2 à 1 % pendant 48 heures (le milieu en présence de l'actif est changé tous les 24 heures). Les cellules sont ensuite lavées, fixées au méthanol froid pendant 4 minutes à 4°C. Les cellules sont incubées en présence d'un anticorps anti-cyto-pankératines monoclonal au 1/200^{ème} pendant 1 heure à température ambiante puis révélées par un second anticorps au 1/50^{ème} pendant 1 heure à température ambiante, couplées à un marqueur fluorochrome « Alexa 488 ». Après montage dans un milieu *ad hoc,* les lames sont observées au microscope à épifluorescence.

Résultats : l'hydrolysat selon l'exemple 2 augmente l'expression des pankératines dans les cellules traitées.

Conclusion : l'hydrolysat selon l'exemple 2 à 1 % augmente l'expression des cytokératines dans les kératinocytes humains normaux. En présence de l'hydrolysat selon l'exemple 2, les cellules sont stimulées et en voie de différenciation.

### Exemple 10 : Etude de l'effet protecteur de l'hydrolysat selon l'exemple 2 sur les cellules cutanées soumises à des rayonnements ultraviolet (UVB)

Le but de cette étude est de déterminer l'effet protecteur de l'hydrolysat selon l'exemple 2 vis-à-vis de kératinocytes humains normaux soumis à un stress par des rayonnements UVB. Pour cela, des tests de viabilité cellulaire ont été réalisés par la technique au MTT.

Protocole : Les kératinocytes humains normaux sont traités avec l'hydrolysat selon l'exemple 2 à 0,5 %, pendant 24 heures, irradiés par des UVB (50 mJ/cm²) puis cultivés encore 24 heures en présence de la même concentration d'hydrolysat selon l'exemple 2. Des contrôles non traités et irradiés sont réalisés dans les mêmes conditions. A la fin de l'expérimentation, les cellules sont incubées dans une solution contenant 0,1 mg/ml de MTT (3-[4, 5-diméthylthiazol-2-yl]-2, 5-diphényltétrazolium, bromure). Ce composé est absorbé par les cellules vivantes puis métabolisé par les enzymes mitochondriales en un composé bleu violet, le formazan, qui sera dosé par spectrophotométrie à 540 nm. La densité optique (D.O.) est alors directement proportionnelle à l'activité enzymatique mitochondriale ainsi qu'au nombre de cellules vivantes.

Résultats: L'évaluation de la viabilité cellulaire par la technique du MTT montre que l'hydrolysat selon l'exemple 2 augmente la viabilité cellulaire après irradiation par les UVB de 16 %.

Conclusion: L'hydrolysat selon l'exemple 2 à 0,5 %, augmente la viabilité cellulaire et protège efficacement les cellules cutanées contre les effets cytotoxiques des rayonnements UVB.

### Exemple 11 : Etude de l'exuression de la HMG-CoA réductase dans des biopsies de peau, en présence de l'hydrolysat selon l'exemple 1

Le but de cette étude est de déterminer l'influence l'hydrolysat selon l'exemple 1 à 0,5 % sur l'expression de la HMG-CoA réductase.

Protocole : Des échantillons de peau humaine sont mis en culture à l'interface air/liquide. L'hydrolysat selon l'exemple 1 à 0,5 % est appliqué typiquement, puis les échantillons sont incubés durant 24 heures ou 48 heures.

Ces échantillons de peau sont ensuite fixés avec du formaldéhyde puis inclus dans la paraffine. Des coupes de 2 à 3 µm sont alors réalisées. L'immunomarquage est effectué après démasquage des sites spécifiques par traitement micro-onde puis incubation dans de la trypsine. L'immunomarquage est réalisé à l'aide d'un anticorps polyclonal de lapin spécifique de la HMG-CoA réductase (Millipore, Upstate), puis d'un anticorps secondaire, couplé à un marqueur fluorescent. Les coupes de peau sont alors examinées au microscope à Epi-fluorescence (Nikon Eclipse E600 microscope).

Résultats : Les observations microscopiques montrent une fluorescence plus forte dans les couches supérieures de l'épiderme des peaux traitées par l'hydrolysat selon l'exemple 1 à 0,5 %, par rapport au contrôle non traité.

Conclusion : L'hydrolysat selon l'exemple 1, stimule l'expression de la HMG-CoA réductase, dans les couches supérieures de l'épiderme.

### Exemple 12 : Etude de l'expression de la HMG-CoA réductase dans les kératinocytes humains normaux, en présence de l'hydrolysat selon l'exemple 2

Le but de cette étude est de déterminer l'influence de l'hydrolysat selon l'exemple 2 sur l'expression de la HMG-CoA réductase dans les kératinocytes humains normaux.

Protocole : Des kératinocytes humains normaux en culture sont traités avec l'hydrolysat selon l'exemple 2 à 0,5 % pendant 24 ou 48 heures (le milieu contenant l'actif est changé toutes les 24 heures). Les cellules sont ensuite lavées, fixées au méthanol froid pendant 4 minutes à 4°C. Les cellules sont incubées en présence d'un anticorps polyclonal de lapin spécifique de la HMG-CoA réductase (Millipore, Upstate), puis d'un anticorps secondaire, couplé à un marqueur fluorescent. Les cellules sont alors examinées au microscope à Epi-fluorescence (Nikon Eclipse E600 microscope).

Résultats : Les observations microscopiques montrent une fluorescence cytoplasmique plus intense dans les cellules traitées par l'hydrolysat selon l'exemple 2 à 0,5 %.

Conclusion : L'hydrolysat selon l'exemple 2 à 0,5 %, stimule l'expression de la HMG-CoA réductase, dans les kératinocytes humains normaux.

### Exemple 13 : Préparation de compositions

### 1 - Crème protection solaire:

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenxoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Hydrolysat selon l'exemple 1 | | 0,5 % |
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

### 2-Crème anti-âge :

| ***Noms commerciaux*** | ***Noms INCI*** | **% *massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Parkii (Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Noms commerciaux*** | ***Noms INCI*** | **% *massique*** |
|---|---|---|
| ***Phase D*** | | |
| TEA | Triethanolamine | 0,18 |

| ***Phase E*** | | |
|---|---|---|
| Hydrolysat selon l'exemple 1 | | 1% |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase Ajuste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors additionnée si souhaité.

### 3 -Crème protectrice de jour :

| ***Noms commerciaux*** | ***Noms INCI*** | **% *massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C10-30Alkyl Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Hydrolysat selon l'exemple 1 | | 2% |

Préparer la phase A et chauffer à 75°C sous agitation. Préparer la phase B en dispersant le carbopol, puis la gomme xanthane sous agitation. Laisser reposer. Chauffer à 75°C.

A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Hydrolysat peptidique activateur de la HMG-CoA réductase, **caractérisé en ce qu'**il provient de l'hydrolyse de plantes choisies parmi l'épeautre (*Triticum monococum*), la pomme de terré (*Solanum tuberosum*), le maïs (*Zea mayz L.),* le pois (*Pisum sativum*) ou de levures du genre *Saccharomyces,* et plus particulièrement de l'espèce *Saccharomyces cerevisiae,* est composé de peptides de poids moléculaire inférieur à 6 kDa et est enrichi en peptide bioactif capable de renforcer la fonction barrière de l'épiderme, ledit peptide bioactif contenant de 4 à 6 acides aminés dont au moins un résidu glycine, un résidu. leucine et un résidu acide glutamique et possédant une séquence de formule générale (I) :
X₁-[Gly,Glu,Leu]-X₂=X₃
dans laquelle,
X₁ est l'alanine, la valine, l'isoleucine ou aucun acide aminé,
X₂ est la serine ou la thréonine,
X₃ est la leucine, l'isoleucine ou aucun acide aminé,
choisie parmi les séquences :
(SEQ ID n°1) Ala-Glu-Gly-Leu-Ser-Ile
(SEQ ID n°2) Leu-Gly-Glu-Ser-Leu
(SEQ ID n°3) Val-Gly-Glu-Leu-Thr
(SEQ ID n°4) Ile-Gly-Glu-Leu-Ser
(SEQ ID n°5) Ala-Gly-Glu-Leu-Ser
(SEQ ID n°6) Gly-Glu-Leu-Thr-Ile
(SEQ ID n°7) Gly-Glu-Leu-Ser.

2. Hydrolysat peptidique selon la revendication précédente, **caractérisé en ce qu'**il contient entre 0,5 et 5,5 g/l de composés de nature peptidique.

3. Composition cosmétique comprenant, dans un milieu physiologiquement acceptable, en tant que principe actif capable de renforcer la fonction barrière dé l'épiderme, l'hydrolysat peptidique défini selon l'une des revendications précédentes, en une quantité représentant de 0,0001 % à 20 % du poids total de la composition, et préférentiellement en une quantité représentant de 0,05 % à 5 % du poids total de la composition.

4. Composition selon la revendication 3, **caractérisée en ce qu'**elle se présente sous une forme adaptée à l'application par voie topique.

5. Composition selon l'une des revendications 3 ou 4, **caractérisée en ce qu'**elle comprend en outre, au moins un autre principe actif favorisant l'action dudit hydrolysat peptidique choisi parmi la vitamine C, la vitamine E, le coenzyme Q10, les extraits polyphénoliques de plantes, les rétinoïdes, le peptide de séquence Gly-Pro-Gln-Gly-Pro-Gln (COLLAXYL®) et l'extrait *d'Artemia* (GP4G®).

6. Composition pharmaceutique comprenant, dans un milieu physiologiquement acceptable, l'hydrolysat peptidique défini selon l'une quelconque des revendications 1 ou 2, à titre de médicament.

7. Utilisation cosmétique d'une quantité efficace d'un hydrolysat peptidique selon l'une des revendications 1 ou 2; pour renforcer la fonction barrière de l'épiderme et stimuler la différenciation épidermique.

8. Utilisation selon la revendication 7, pour prévenir et lutter contre les signes cutanés du vieillissement et du photo-vieillissement.

9. Utilisation selon la revendication 7, pour protéger la peau des agressions extérieures.

10. Procédé de traitement cosmétique destiné à prévenir ou traiter les manifestations cutanées du vieillissement et/ou du photo-vieillissement, **caractérisé en ce que** l'on applique topiquement sur la peau ou les phanères à traiter, une composition comprenant une quantité efficace d'hydrolysat peptidique tel que défini selon l'une des revendications 1 ou 2.

## Patentansprüche

1. Aktivierendes Peptidhydrolysat von HMG-CoA-Reduktase, **dadurch gekennzeichnet, dass** es aus der Hydrolyse von Pflanzen hervorgeht, die aus Dinkel *(Triticum monococum),* Kartoffel *(Solanum tuberosum),* Mais *(Zea mayz L.*), Erbse *(Pisum sativum)* oder Hefen der Gattung *Saccharomyces* und insbesondere der Spezies *Saccharomyces cerevisiae* ausgewählt sind, sich aus Peptiden mit einem Molekulargewicht von weniger als 6 kDa zusammensetzt und mit bioaktivem Peptid angereichert ist, das die Barrierefunktion der Epidermis verstärken kann, wobei das besagte bioaktive Peptid 4 bis 6 Aminosäuren enthält, darunter mindestens einen Glycinrest, einen Leucinrest und einen Glutaminsäurerest, und eine Sequenz der folgenden allgemeinen Formel (I) aufweist:
X₁-[Gly, Glu, Leu]-X₂-X₃,
in der
X₁ Alanin, Valin, Isoleucin oder keine Aminosäure ist,
X₂ Serin oder Threonin ist,
X₃ Leucin, Isoleucin oder keine Aminosäure ist,
und die aus den folgenden Sequenzen ausgewählt ist:
(SEQ ID Nr. 1) Ala-Glu-Gly-Leu-Ser-Ile
(SEQ ID Nr. 2) Leu-Gly-Glu-Ser-Leu
(SEQ ID Nr. 3) Val-Gly-Glu-Leu-Thr
(SEQ ID Nr. 4) Ile-Gly-Glu-Leu-Ser
(SEQ ID Nr. 5) Ala-Gly-Glu-Leu-Ser
(SEQ ID Nr. 6) Gly-Glu-Leu-Thr-Ile
(SEQ ID Nr. 7) Gly-Glu-Leu-Ser.

2. Peptidhydrolysat nach dem vorhergehenden Anspruch, **dadurch gekennzeichnet, dass** es zwischen 0,5 und 5,5 g/l natürlicher Peptidverbindungen enthält.

3. Kosmetische Zusammensetzung, die in einem physiologisch akzeptablen Medium als Wirkstoff, der die Barrierefunktion der Epidermis verstärken kann, das in einem der vorhergehenden Ansprüche definierte Peptidhydrolysat in einer Menge umfasst, die 0,0001 % bis 20 % des Gesamtgewichts der Zusammensetzung ausmacht, und vorzugsweise in einer Menge umfasst, die 0,05 % bis 5 % des Gesamtgewichts der Zusammensetzung ausmacht.

4. Zusammensetzung nach Anspruch 3, **dadurch gekennzeichnet, dass** sie in einer Form dargereicht wird, die für eine topische Anwendung geeignet ist.

5. Zusammensetzung nach einem der Ansprüche 3 oder 4, **dadurch gekennzeichnet, dass** sie außerdem mindestens einen anderen Wirkstoff umfasst, der die Wirkung des besagten Peptidhydrolysats begünstig und aus Vitamin C, Vitamin E, Coenzym Q10, polyphenolischen Pflanzenextrakten, Retinoiden, dem Peptid mit der Sequenz Gly-Pro-Gln-Gly-Pro-Gln (COLLAXYL®) und dem *Artemia-*Extrakt (GP4G®) ausgewählt ist.

6. Pharmazeutische Zusammensetzung, die in einem physiologisch akzeptablen Medium das in einem der Ansprüche 1 oder 2 definierte Peptidhydrolysat umfasst, als Arzneimittel.

7. Kosmetische Verwendung einer wirksamen Menge eines Peptidhydrolysats nach einem der Ansprüche 1 oder 2 zum Verstärken der Barrierefunktion der Epidermis und zum Stimulieren der epidermalen Differenzierung.

8. Verwendung nach Anspruch 7 zum Verhindern und Bekämpfen von Zeichen der Alterung und der Lichtalterung der Haut.

9. Verwendung nach Anspruch 7 zum Schützen der Haut vor äußeren Einflüssen.

10. Verfahren zur kosmetischen Behandlung, die dazu vorgesehen ist, Manifestationen der Alterung und/oder der Lichtalterung der Haut zu bekämpfen oder zu behandeln, **dadurch gekennzeichnet, dass** eine Zusammensetzung, die eine wirksame Menge des wie in einem der Ansprüche 1 oder 2 definierten Peptidhydrolysats umfasst, topisch auf die zu behandelnde Haut oder die zu behandelnden Hautanhangsgebilde aufgebracht wird.

## Claims

1. Peptide hydrolysate for activating HMG-CoA reductase, **characterised in that** it is obtained from the hydrolysis of plants chosen from einkorn (*Triticum monococcum*), potato *(Solanum tuberosum),* maize *(Zea mays L.),* pea *(Pisum sativum)* or yeasts of the *Saccharomyces* genus, and particularly of the species *Saccharomyces cerevisiae,* is composed of peptides having a molecular weight less than 6 kDa and is enriched with bioactive peptide capable of reinforcing the barrier function of the epidermis, said bioactive peptide containing 4 to 6 amino acids including at least one glycine residue, one leucine residue and one glutamic acid residue and having a sequence according to general formula (I):
X₁-[Gly,Glu,Leu]-X₂-X₃
wherein,
X₁ is alanine, valine, isoleucine or no amino acid,
X₂ is serine or threonine,
X₃ is leucine, isoleucine or no amino acid,
chosen from the sequences:
(SEQ ID No. 1) Ala-Glu-Gly-Leu-Ser-Ile
(SEQ ID No. 2) Leu-Gly-Glu-Ser-Leu
(SEQ ID No. 3) Val-Gly-Glu-Leu-Thr
(SEQ ID No. 4) Ile-Gly-Glu-Leu-Ser
(SEQ ID No. 5) Ala-Gly-Glu-Leu-Ser
(SEQ ID No. 6) Gly-Glu-Leu-Thr-Ile
(SEQ ID No. 7) Gly-Glu-Leu-Ser.

2. Peptide hydrolysate according to the above claim, **characterised in that** it contains between 0.5 and 5.5 g/l of peptide compounds.

3. Cosmetic composition comprising, in a physiologically acceptable medium, as an active substance capable of reinforcing the barrier function of the epidermis, the peptide hydrolysate defined according to any of the above claims, in a quantity representing from 0.0001% to 20% of the total weight of the composition, and preferentially in a quantity representing from 0.05% to 5% of the total weight of the composition.

4. Composition according to claim 3, **characterised in that** it is presented in a form suitable for topical application.

5. Composition according to any of claims 3 or 4, **characterised in that** it further comprises at least one further active substance promoting the action of said peptide hydrolysate chosen from vitamin C, vitamin E, coenzyme Q10, polyphenolic plant extracts, retinoids, the peptide having the sequence Gly-Pro-Gln-Gly-Pro-Gln (COLLAXYL®) and *Artemia* extract (GP4G®).

6. Pharmaceutical composition comprising, in a physiologically acceptable medium, the peptide hydrolysate defined according to any one of claims 1 or 2, as a medicinal product.

7. Cosmetic use of an effective quantity of a peptide hydrolysate according to any of claims 1 or 2, for reinforcing the barrier function of the epidermis and stimulating epidermal differentiation.

8. Use according to claim 7, for preventing and combating the signs of skin ageing and photo-ageing.

9. Use according to claim 7, for protecting the skin against external attacks.

10. Cosmetic treatment method intended to prevent or treat the skin signs of ageing and/or photo-ageing, **characterised in that** a composition comprising an effective quantity of peptide hydrolysate according to any of claims 1 or 2 is applied topically on the skin or skin appendages to be treated.


### SEQUENCE LISTING

<110> ISP Investments INC.
<120> COMPOSITION COSMÉTIQUE ET/OU PHARMACEUTIQUE COMPRENANT UN HYDROLYSAT PEPTIDIQUE CAPABLE DE RENFORCER LA FONCTION BARRIERE
<130> Bv PCT 09-122
<150> FR 09 01822
<151> 2009-04-15
<160> 7
<170> PatentIn version 3.3
<210> 1
   <211> 6
   <212> PRT
   <213> Plant OR Saccharomyces cerevisiae
<400> 1
   Ala Glu Gly Leu Ser Ile
   1 5
<210> 2
   <211> 5
   <212> PRT
   <213> Plant OR Saccharomyces cerevisiae
<400> 2
   Leu Gly Glu Ser Leu
   1 5
<210> 3
   <211> 5
   <212> PRT
   <213> Plant OR Saccharomyces cerevisiae
<400> 3
   Val Gly Glu Leu Thr
   1 5
<210> 4
   <211> 5
   <212> PRT
   <213> Plant OR Saccharomyces cerevisiae
<400> 4
   Ile Gly Glu Leu Ser
   1 5
<210> 5
   <211> 5
   <212> PRT
   <213> Plant OR saccharomyces cerevisiae
<400> 5
   Ala Gly Glu Leu Ser
   1 5
<210> 6
   <211> 5
   <212> PRT
   <213> Plant OR Saccharomyces cerevisiae
<400> 6
   Gly Glu Leu Thr Ile
   1 5
<210> 7
   <211> 4
   <212> PRT
   <213> Plant OR Saccharomyces cerevisiae
<400> 7
   Gly Glu Leu Ser
   1